# EUROPÄISCHE PATENTSCHRIFT

(11) **EP 0 532 144 B1**
(45) Veröffentlichungstag und Bekanntmachung des Hinweises auf die Patenterteilung: **24.02.1999**
(21) Anmeldenummer: 92250258.8
(22) Anmeldetag: 14.09.1992
(51) Int. Cl.: A61N 1/365

(54) **Herztherapiesystem**
Cardiac therapy system
Système de thérapie cardiaque

(30) Priorität: 12.09.1991 DE 4130595
(43) Veröffentlichungstag der Anmeldung: 17.03.1993
(73) Patentinhaber: BIOTRONIK Mess- und Therapiegeräte GmbH & Co Ingenieurbüro Berlin, D-12359 Berlin (DE)
(72) Erfinder: Schaldach, Max, Prof. Dr., W-8520 Erlangen (DE)
(74) Vertreter: Christiansen, Henning, Dipl.-Ing.

(56) Entgegenhaltungen:
- US-A- 4 719 921
- US-A- 4 733 667

## Beschreibung

Die Erfindung betrifft ein Herztherapiesystem der im Oberbegriff des Anspruchs 1 angegebenen Art.

Es ist bei Herzschrittmachern bekannt, aus dem Körper des Patienten aufgenommene Signale, die für die äußere Belastung oder die Belastung von Körperorganen kennzeichnend sind, zur Steuerung der Herzstimulation heranzuziehen. Darüber hinaus ist es bekannt, solche Signale zu benutzen, welche ausschließlich ein Maß für den keardiovaskulären Herzleistungsbedarf beinhalten und damit eine Information des autonomen Nervensystems (ANS) bilden.

Aus US-A-4 733 667 ist es bekannt, die Zeitableitung der intrakardialen Impedanz, aus der ein Maß für den kardiovaskulären Leistungsbedarf bildende Aktivitätsignale des ANS herleitbar sind, zur physiologischen Steuerung eines Herzschrittmachers zu nutzen.

Es hat sich herausgestellt, daß eine Anzahl von Herzerkrankungen nicht allein durch schrittmachende Stimulationsimpulse behandelt werden kann.

Der Erfindung liegt die Aufgabe zugrunde, bei einem Herztherapiesystem der eingangs genannten Gattung die Behandlungsmöglichkeiten Zu erweitern.

Diese Aufgabe wird mit den kennzeichnenden Merkmalen des Anspruchs 1 gelöst.

Die Erfindung beruht auf der Erkenntnis, daß die Informationen des autonomen Nervensystems nicht nur für die Erzeugung von Stimulationsimpulsen, sondern auch für weitere am Herzen angreifende Therapiemaßnahmen günstige Eingangsgrößen bilden.

Zur Erzeugung weiterer diese Therapiemaßnahmen beeinflussendenr Signale eignen sich die Informationen des autonomen Nervensystems in besonderer Weise, weil sie unmittelbar ein Maß für den internen keardiovaskulären Herzleistungsbedarf bilden und nicht - wie beispielsweise die von der Aktivität des Patienten gesteuerten Schrittmacher - sich über die Belastung aus einer externen Größe herleiten oder mit weiteren Belastungs- bzw. Herz-Kreislaufgrößen vernetzt bzw. von diesen abhängig sind.

Eine Analyse der Kreislaufhämodynamik ergibt, daß die kardiovaskuläre Leistung in der Form einer Regelschaltung (negative feedback controlled system) ausreichend beschrieben werden kann. Der durchschnittliche arterielle Blutdruck ist die kontrollierte Größe und der Herzleistungsbedarf die kontrollierende Variable. Unter physiologischen Bedingungen funktioniert die kurzfristige °Steuerung des mittleren arteriellen Blutdrucks gut. Alle Anforderungen an das kardiovaskuläre System werden im Gleichgewicht gehalten, und es wird daher eine zuverlässige Perfusion des Gewebes erreicht.

Die meisten Anforderungen an das kardivaskuläre System beeinflussen den peripheren Gefäßwiderstand. Die relevantesten dieser Anforderungen sind: körperliche Betätigung, Haut- und die Kerntemperatur des Körpers, der Säurespiegel, das Gleichgewicht und die Körperhaltung. Eine der Anforderungen beeinflußt sowohl die resistiven als auch die kapazitiven Eigenschaften der Peripherie durch Veränderungen in der Verengung der Arteriola oder Venula. Die sekundären Wirkungen sind Veränderungen im Gefäßwidderstand, im Blutdruck, im Venenrückfluß und in der Herzleistung. Beispielsweise kann sich der Gefäßwiderstand während einer körperlichen Betätigung um das fünffache verändern. Um den Blutdruck konstant zu halten, müssen der Venenrückfluß und die Herzleistung entsprechend mit entgegengesetzter Wirkung verändert werden.

Der Herzleistungsbedarf ist hier die kontrollierende Größe. Sie ist das Produkt der Herzfrequenz und des Schlagvolumens. Veränderungen Herzleistungsbedarfs stehen im umgekehrten Verhältnis zu den Veränderungen der Gefäßwiderstandes mit dem Ergebnis, daß der mittlere arterielle Blutdruck im wesentlichen konstant gehalten wird. Unter physiologischen Bedingungen wird die Anpassung des Herzleistungsbedarfs an die Veränderungen des Gefäßwiderstandes von einem (closed-loop feedback) mit Barorezeptoren im Aortenbogen und im Sinus caroticus, die als Drucksensoren arbeiten, durchgeführt. Die Ausgangssignale dieser Barorezeptoren werden den medullären kardiovaskulären Zentren zugeführt. Von diesen Zentren werden Ausgangssignale zum Herz über den Sympathikus oder Parasympathikus geleitet.

Der Sympathikus reguliert die Sinusfrequenz und das ventrikuläre Schlagvolumen. Der Parasympathikus dagegen beeinflußt überwiegend die Herzfrequenz. Die Kombination der chronotropischen und der inotropischen Faktoren führt zusammen zur Steuerung der Herzleistung. Andere Variable, die das Herz aufgrund der gleichen externen Pfade beeinflussen, sind u.a.: Schmerz, Emotion und physiologischer Streß.

Bei Patienten mit einer chronotropischen Insuffizienz wird der Rückkopplungsmechanismus, mit dem die Herzfrequenz über den Sinusknoten reguliert wird, unterbrochen. Als Folge können diese Patienten nur durch eine Änderung des Schlagvolumens auf Belastungen reagieren. Die mögliche Schlagvolumenveränderung ist aber bei einer körperlichen Betätigung sehr gering.

Die Stimulationtherapie bietet bisher verschiedene Strategien, um dem Einfluß einer Belastung des Kreislaufs entgegenzuwirken.

Das erfindungsgemäße Herztherapiesystem mit Steuerung durch ein Maß für den kardiovaskulären Leistungsbedarf bildende Signale des autonomen Nervensystems (ANS) wird bevorzugt in Ergänzung zu einer herkömmlichen Herzschrittmacherschaltung benutzt.

In Abhängigkeit von der Intensität und wahlweise auch Zeit der im Körper des Patienten aufgenommenen ANS-Signale werden Steuersignale erzeugt, mittels derer über eine Nervenstimulation der Gefäßwiderstand in Anpassung an den intrakardialen Leistungsbedarf veränderbar ist. Auf diese Weise lassen sich Gefäßwiderstand und Herzleistung aneinander anpassen. Bei vielen Kreislaufkrankheiten wie Durchblutungsstörungen oder Gefäßspasmus kann auf eine chemische Therapie verzichtet werden. Durch die Stimulationsbehandlung der für den jeweiligen Gefäßbereich maßgeblichen Nervenleiter wird der Gefäßwiderstand dem aktuellen physiologischen Leistungsbedarf angepaßt, so daß dieser den jweiligen cardiovaskulären Erfordernissen entspricht. Durch die Möglichkeit, kurzfristiger Änderungen ist die hier beschriebene Neurostimulation chemischen Therapiemaßnahmen überlegen.

Darüberhinaus können auch antiarrhythmische Stimulationsimpulse in Abhängigkeit von und in Synchronität zu ANS-Signalen ausgelöst bzw. synchronisiert werden. Diese Signale weisen einen Informationsgehalt auf, der sie dazu befähigt, tachycardieterminierend bzw. defibrillierend zu wirken, da die Signale des autonomen Nervensystem sozusagen die "Grundinformation" für die Synchronität der Herztätigkeit mit den diese steuernden Grundsysteme des Körpers beinhalten.

Entsprechend läßt sich durch die Signale des autonomen Nervensystems auch ein Pumpassistsystem aktivieren bzw. synchronisieren. Hierbei wird die Tatsache ausgenutzt, daß die benötigte fehlende Pumpleistung bei entsprechender Insuffizienz des Herzens durch das Assistsystem erbracht werden muß, wobei die benötigte Gesamtleistung direkt dem aufgrund von ANS ermittelten cardiovaskulären Leistungsbedarf entspricht.

Die von den ANS-Signalen abgeleiteten Signale lassen sich nach Bedarf vorteilhaft mit Steuersignalen in ihrer Auswirkung logisch verknüpfen, welche im Körper des Patienten aufgenommen werden und ein Maß für die äußere körperliche Belastung des Patienten oder die Belastung von Körperorganen bilden. Hierbei werden die genannten Steuersignale vorzugsweise in Abhängigkeit von jeweils vorgebbaren Ereignismustern, d.h. vom Auftreten der ANS-Signale innerhalb vorgegebener Zeitfenster, ihrer Häufigkeit und/oder Intensität sowie gegebenenfalls der weiteren Signale und/oder externer Programmiersignale und/oder der früher auf diese Weise erzeugten Steuersignale selbst korreliert.

Eine zentrale, für den kardiovaskulären Bedarf maßgebliche und gut reproduzierbare Größe des autonomen Nervensystems (ANS) stellt die regionale effektive Steigung (ROI) der intrakardialen Impedanz des rechten Ventrikels dar. Hierbei wird bevorzugt das Intervall mit der regelmäßig größten Änderung bei isovolumetrischer Kontraktion und hiervon wiederum der Bereich mit der größten Veränderung bei Belastungsänderungen des Patienten ausgewählt. Damit hat das ermittelte Signal brauchbare Eigenschaften, die dazu führen, daß es sich bevorzugt als Steuersignal für die das Herzzeitvolumen bestimmenden Größen des Herzschrittmachers eignet.

Die regionale effektive Steigung steht im umgekehrten Verhältnis zur Myokardkontraktilität. Je größer die Kontraktilität, desto schneller muß sich der notwendige Druck zur Öffnung der ventrikulären Herzklappen aufbauen kann.

Die ANS bildet dabei eine von weiteren Einflußgrößen weitgehend unabhängige, für den Herzleistungsbedarf repräsentative, autonome Nerveninformation. Sie zeigt inotropisches Verhalten als Anzeichen für den cardiovaskulären Bedarf als Steuersignal für die Wiederherstellung des chronotropischen Verhaltens und stellt eine für die Modulation der Kontraktilität maßgebliche Größe dar. Es hat sich gezeigt, daß eine ausreichende Auflösung mit einer im Ventrikel angeordneten unipolaren Elektrode erzielt werden kann, wobei das Herzschrittmachergehäuse als Gegenelektrode dient.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein vorteilhaftes Ausführungsbeispiel der Erfindung im Blockschaltbild,
Figur 2 einen Teil des in Figur 1 wiedergegbenen Ausführungsbeispiels im Detail sowie
Figur 3 eine schematische Darstellung zur Neurostimulation in Anwendung der Erfindung.

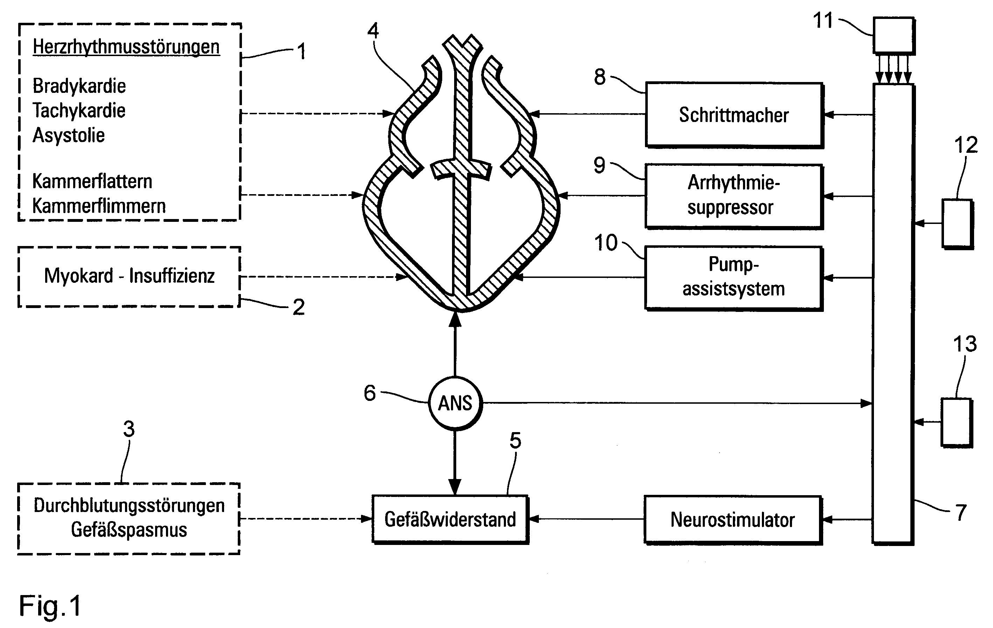

Bei dem in Figur 1 dargestellten Ausführungsbeispiel ist im linken Teil der Figur in Blöcken 1 bis 3 eine Reihe von Herz-Kreislauf-Krankheiten angegeben, welche auf das kardio-vaskuläre System, repräsentiert durch das Herz 3 und das Gefäßsystem 5, welches durch seinen Widerstand im Kreislauf charakterisiert ist, einwirken.

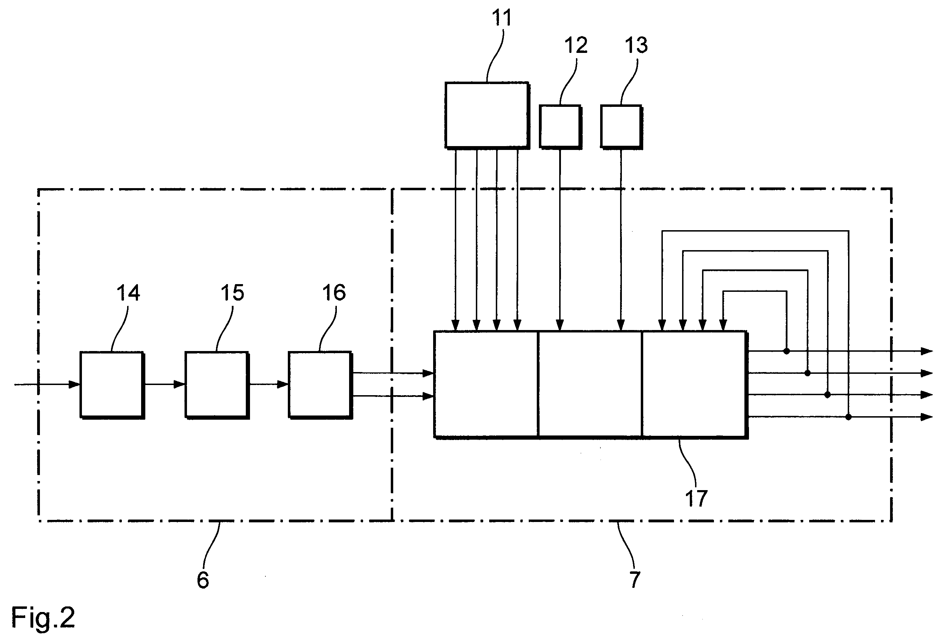

Abgeleitet wird die Größe ANS als kreislaufwirksame Größe des autonomen Nervensystems in einem Block 6. Die ermittelte, für den keardiovaskulären Bedarf hinsichtlich Intensität und Synchronität maßgebliche, Größe wird in einer Steuerschaltung 7 weiterverarbeitet, welche in Figur 2 näher dargestellt ist.

An die Steuerschaltung 7 ist ein Schrittmacher 8 zur Herzstimulation und ein Arrhythmiesuppressor 9 zur Defibrillation bzw. Tachycardieterminierung angeschlossen.

Ein Pumpassistsystem 10 ersetzt den Herzmuskel mindestens teilweise bei auftretender Insuffizienz der Punpaktivität.

Mittels einer externen Programmierschaltun 11 lassen sich die internen Signalverknüpfungen der Steuerschaltung 7 beeinflussen, wobei gegebenenfalls externe Signalaufnehmer 12 und 13 mit berücksichtigt werden, welche innerhalb bzw. außerhalb des Körpers des Patienten aufgenommene Signale erfassen, die ein Maß für die äußere körperliche Belastung oder aber für die Belastung einzelner Organe des Patienten bilden, welche wiederum von der äußeren körperlichen Belastung abhängig ist.

Bei dem in Figur 2 im Detail wiedergegebenen Blöcken 6 und 7 der Blockschaltung gemäß Figur 1 handelt es sich im Falle des Blocks 6 um die Gewinnung eines Signals mit der Information des autonomen Nervensignals.

Hierbei wird in einem ersten Block 14 vom Herzen die intrakardiale Impedanz und in einem Block 15 als Aktivitätsgröße des autonomen Nervensystems die regionale effektive Steigung (ROI) der intrakardialen Impedanz im Bereich des rechten Ventrikels ermittelt. In einem weiteren nachgeschalteten Block 16 wird die Erfassung der Aktivitätsgröße jeweils auf den Bereich maximaler von der Aktivität des Patienten abhängiger Änderungen beschränkt. Die beiden Ausgangssignalleitungen des Blocks 16 repräsentieren dabei jeweils die die Intensität bzw. die zeitliche Lage im Herzzeitintervall (Synchronität) betreffenden Informationen der Aktivitätsgröße des ANS-Signals.

In dem Block 17 erfolgt die logische Verknüpfung der der Eingangssignale des Therapiesystems zu den benötigten Ausgangssignalen.

Dabei werden in Abhängigkeit von der Intensität und wahlweise zusätzlich Zeit der im Körper des Patienten aufgenommenen ANS-Signale Steuersignale erzeugt, mittels derer über eine Nervenstimulaticn der Gefäßwiderstand in Anpassung an den intrakardialen Leistungsbedarf verändere wird, antiarrhythmische Stimulationsimpulse ausgelöst und/oder synchronisiert werden bzw. ein Pumpassistsystem aktiviert und/oder synchronisiert wird. Hierbei bildet der Block 17 einen Speicher für Steuersequenzen, welche in Abhängigkeit von den Eingangssignalen abgerufen werden können. Die Eingangssignale bilden dabei in digitalisierter Form "Adressen" für den Speicher, so daß - im Extremfall - für jede Eingangssignalkombination eine entsprechende Ausgangssignalsequenz für die jeweils anzusteuernden Blöcke vorrätig ist.

Dabei werden die Steuersignale in Abhängigkeit von weiteren Signalen ausgelöst, welche im Körper des Patienten aufgenommen werden und ein Maß für die äußere körperliche Belastung des Patienten oder die Belastung von Körperorganen bilden. Diese Steuersignale werden in Abhängigkeit von jeweils vorgebbaren Ereignismustern, d.h. vom Auftreten der ANS-Signale innerhalb vorgegebener Zeitfenster, ihrer Häufigkeit und/oder Intensität sowie gegebenenfalls der weiteren Signale und/oder externer Programmiersignale und/oder der früher auf diese Weise erzeugten Steuersignale selbst erzeugt.

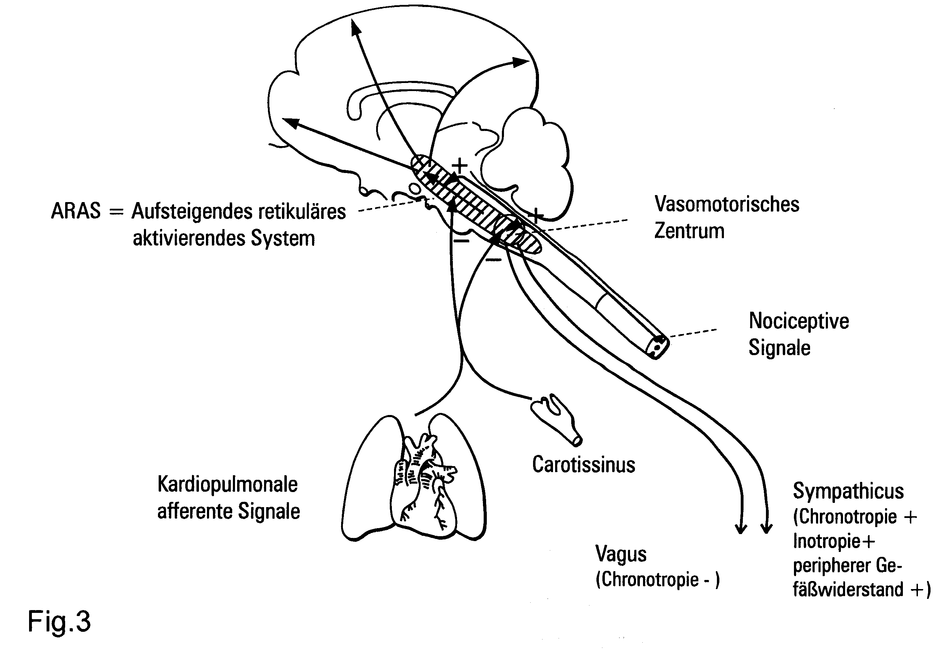

In Figur 3 ist prinzipiell die Beeinflussung der kreislaufwirksamen ANS-Aktivität durch nociceptive Reize wiedergegeben. Dabei wird durch die im Herzen erfaßte ANS-Aktivität die entsprechende Aktivität in anderen Bereichen des Kreislaufsystems gezielt beeinflußt, so daß hier eine Korrkektur im Sinne der der im Herzen aufgenommenen ANS-Aktivität erfolgen kann.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Herztherapiesystem mit Steuerung durch ein Maß für den kardiovaskulären Leistungsbedarf bildende Aktivitätssignale des autonomen Nervensystems ANS (6), insbesondere in Ergänzung zu einer herkömmlichen Herzschrittmacherschaltung (8, 9),
**dadurch gekennzeichnet**,
daß in Abhängigkeit von der Intensität der im Körper des Patienten aufgenommenen ANS-Signale Steuersignaie erzeugt werden, mittels derer
über eine Nervenstimulation (11) der Gefäßwiderstand (5) in Anpassung an den intrakardiaien Leistungsbedarf verändert wird
und/oder
antiarrhythmische Stimuiationsimpulse ausgelöst und/oder synchronisiert werden
und/oder
ein Pumpassistsystem (10) aktiviert und/oder synchronisiert wird.

2. Herztherapiesystem nach Anspruch 1, **dadurch gekennzeichnet**, daß die Steuersignale weiterhin in Abhängigkeit von der Zeit der im Körper aufgenommenen ANS-Signale erzeugt werden.

3. Herztherapiesystem nach einem der vorangehenden Ansprüche, **dadurch gekennzeichnet**, daß die Steuersignale in Abhängigkeit von weiteren Signalen ausgelöst werden, welche im Körper des Patienten aufgenommen werden und ein Maß für die äußere körperliche Belastung des Patienten oder die Belastung von Körperorganen bilden.

4. Herztherapiesystem nach einem der vorangehenden Ansprüche, **dadurch gekennzeichnet**, daß die Steuersignale in Abhängigkeit von jeweils vorgebbaren Ereignismustern, d.h. vom Auftreten der ANS-Signale innerhalb vorgegebener Zeitfenster, ihrer Häufigkeit und/oder Intensität sowie gegebenenfalls weiterer Signale und/oder externer Programmiersignale und/oder der früher auf diese Weise erzeugten Steuersignale selbst erzeugt werden.

5. Herztherapiesystem nach einem der vorangehenden Ansprüche, **dadurch gekennzeichnet**, daß Aktivitätssignale des autonomen Nervensystems (6) aus der regionalen effektiven Steigung ROI der intrakardialen Impedanz im Bereich des rechten Ventrikels gewonnen werden.

6. Herztherapiesystem nach Anspruch 5, **dadurch gekennzeichnet**, daß die Gewinnung der Aktivitätssignale im Bereich maximaler von der Aktivität des Patienten abhängiger Änderungen erfolgt.

7. Herztherapiesystem nach einem der vorangehenden Ansprüche, **dadurch gekennzeichnet**, daß mittels der Nervenstimulation und/oder den antiarrhythmischen Signalen die ANS-Aktivität durch nociceptive Reize beeinflußt wird.

## Claims

1. A heart therapy system controlled by activity signals of the autonomous nervous system ANS (6) which provide a measurement of cardiovascular performance, especially as a supplement to a conventional heart pacemaker circuit (8, 9), characterised in that depending on the intensity of the ANS signals in the patient's body, control signals are generated, by means of which
the vessel resistance (5) is varied via a nerve stimulation (11) in accordance with the intracardiac performance
and/or
antiarrythmic stimulation pulses are triggered and/or synchronised
and/or
a pump assist system (10) is activated and/or synchronised.

2. A heart therapy system according to claim 1, characterised in that the control signals are furthermore generated dependent on the time of the ANS signals recorded in the body.

3. A heart therapy system according to one of the preceding claims, characterised in that the control signals are triggered dependent on further signals which are recorded in the patient's body and form a measurement for the external load on the patient's body or the load on the body's organs.

4. A heart therapy system according to one of the preceding claims, characterised in that the control signals are generated in dependence on respectively pre-definable event patterns, i.e. on the occurrence of ANS signals within specified time windows, their rate of occurrence and/or intensity and possibly other signals and/or external programming signals and/or control signals which were themselves generated earlier in this way.

5. A heart therapy system according to one of the preceding claims, characterised in that activity signals of the autonomous nervous system (6) are gained from the regional effective increase ROI of the intracardiac impedance in the area of the right ventricle.

6. A heart therapy system according to claim 5, characterised in that the production of activity signals occurs in the area of maximum variations dependent on the activity of the patient.

7. A heart therapy system according to one of the preceding claims, characterised in that the ANS activity is influenced by nociceptive stimuli via nerve stimulation and/or antiarrhythmic signals.

## Revendications

1. Système de thérapie cardiaque par commande par des signaux d'activité du système nerveux autonome ANS (6) constituant une mesure pour le besoin de puissance cardiovasculaire, en particulier en complément d'un circuit de stimulateur cardiaque classique (8, 9),
caractérisé
en ce qu'en fonction de l'intensité des signaux ANS reçus dans le corps du malade, des signaux de commande sont générés, au moyen desquels
par l'intermédiaire d'une stimulation nerveuse (11), la résistance des vaisseaux (5) est modifiée en adaptation au besoin de puissance intracardiaque
et/ou
des impulsions de stimulation anti-arythmiques sont déclenchées et/ou synchronisées
et/ou
un système d'assistance à pompe (10) est activé et/ou synchronisé.

2. Système de thérapie cardiaque selon la revendication 1, caractérisé en ce que les signaux de commande sont en outre générés en fonction de l'instant des signaux ANS reçus dans le corps.

3. Système de thérapie cardiaque selon l'une quelconque des revendications précédentes, caractérisé en en que les signaux de commande sont déclenchés en fonction d'autres signaux qui sont reçus dans le corps du malade et constituent une mesure de la charge corporelle extérieure du malade ou de la charge d'organes corporels.

4. Système de thérapie cardiaque selon l'une quelconque des revendications précédentes, caractérisé en ce que les signaux de commande sont eux-mêmes générés en fonction de modèles d'événements pouvant être simulés, c'est-à-dire par l'apparition de signaux ANS à l'intérieur d'une fenêtre temporelle prescrite, de leur fréquence et/ou de leur intensité, ainsi éventuellement que d'autres signaux et/ou signaux de programmation extérieurs et/ou des signaux de commande générés de cette manière antérieurement.

5. Système de thérapie cardiaque selon l'une quelconque des revendications précédentes, caractérisé en ce que des signaux d'activité du système nerveux autonome (6) sont obtenus à partir de l'accroissement effectif régional ROI de l'impédance intracardiaque à proximité du ventricule droit.

6. Système de thérapie cardiaque selon la revendication 5, caractérisé en ce que l'obtention des signaux d'activité s'effectue dans la plage de variations maximales en fonction de l'activité du malade.

7. Système de thérapie cardiaque selon l'une quelconque des revendications précédentes, caractérisé en ce qu'au moyen de la stimulation nerveuse et/ou des signaux anti-arythmiques, l'activité ANS est influencée par des stimulations nociceptives.
